# EUROPEAN PATENT APPLICATION

(11) **EP 1 586 578 A2**
(43) Date of publication of application: **19.10.2005**
(21) Application number: 05005183.8
(22) Date of filing: 09.03.2005
(51) Int. Cl.: C07F 15/00

(54) **Method of removing allyl series protecting group using novel ruthenium complex and method of synthesizing allyl ethers**

(30) Priority: 10.03.2004 JP 2004068217; 02.02.2005 JP 2005027096
(71) Applicant: NAGOYA UNIVERSITY, Chikusa-ku Nagoya City Aichi Prefecture (JP)
(72) Inventor: Kitamura, Masato, Nagoya-shi Aichi-ken (JP); Tanaka, Shinji, Nagoya-shi Aichi-ken (JP); Saburi, Hajime, Nagoya-shi Aichi-ken (JP)
(74) Representative: Heusler, Wolfgang

(57) **Abstract**

A cyclopentadienyl ruthenium (II) complex or (iv) complex having an α-imino acid type ligand or an α-amino acid type ligand.

## Description

The present invention relates to a technology for removing allyl group useful as a protecting group of, for example, hydroxyl group by using a novel ruthenium complex. The present invention also relates to a technology for synthesizing allyl ethers by means of catalytic dehydration type reaction of alcohols using the ruthenium complex.

The protecting group plays a crucial role in the multistep syntheses of organic molecules having a variety of functional groups. In particular, the allyl group is useful as a protecting group of the hydroxyl group because of, for example, its simplicity of structure and its stability relative to the acid and base, and the allyl ether has become a focus of attention as a protected compound.

Demands for the simplification of the synthesizing process, for the economic advantage, and for the environmental harmony are being enhanced in addition to the demands for the high reaction rate and for the high functional group selectivity in respect of the removal of the protecting group that is carried out in the recent technology of the organic synthesis. Various catalytic removing methods and non-catalytic removing methods are reported in respect of the removal of the allyl group in, for example, J. Cunningham, R. Ciggs. C.D. Warren, Tetrahedron Lett. 1964, p1191-1196"; K. C. Nicolaou, C. W. Hummel, N. J. Bockovich, C. H. Wong, J. Chem. Soc., Chem. Commun. 1991, 870-872; T. Taniguchi, K. Ogasawara, Angew. Chem, Int. Ed. 1998, 37, 1136-1137; and A. Dahlen, A. Sundgren, M. Lahmann, S. Oscarson, G. Hilmersson, Org. Lett. 2003, 5, 4085-4088.

However, any of the removing methods disclosed in the publications exemplified above requires a multistep reaction and the addition of an acid, a base and a reducing agent for the removal of the allyl group. Such being the situation, the technology fully capable of coping with the demands noted above has not yet been established.

On the other hand, in respect of the synthesis of allyl ether useful as a protected compound of a hydroxyl group, most of them are dependent upon Williamson type ether synthesis method (see, for example, Williamson, A. W, J, Chem. Soc. 1852, 4,229.). Such a synthesis method has the advantage that target allyl ether can be synthesized with a high chemical yield. However, it is necessary to convert alcohol into metal alkoxide or alkyl-halide, and to convert allyl alcohol into a corresponding halide or alkoxide, and the atomic efficiency is low such that an equivalent metal salt is produced as a by-product by activating the substrate, which becomes a double loss. Moreover, the E factor ([(all of the amount of substance used in synthesizing of chemical substances) -(the amount of substance sold as a product)]/(the amount of substance sold as a product)) is also high. Furthermore, since the reaction system becomes a significant basicity, the chemical selectivity is also reduced.

For the development of an ideal method of synthesizing allyl ether, a dehydration method using an acid catalyst (see, for example, Moffett, E. J. Am. Chem. Soc. 1934, 56, 2009; and Senderens, M. J.-B. Compt. Rend. 1925, 181, 698-701.), an oxymetallation and dehydroxymetallation method using Hg (II), Pd (II), or Cu (II) catalyst (see, for example, Watanabe, W. H.; Conlon, L. E.; Hwa, J. C. H. J. Org. Chem. 1958, 23, 1666-1668; Dumlao, C. M.; Francis, J. W.; Henry, P. M. Organometallics 1991, 10, 1400-1405; and Oguchi, W.; Uchida, H. WO Patent 03/106024, 2003.) and a variety of catalytic methods based on the π allyl mechanism (see, for example, Jpn. Pat. Appln. KOKAI Publication No. 05-306246) have been so far reported. However, the respective methods have the problems such that the chemical yield is low, the catalytic efficiency is low, allyl alcohol must be used excessively, dialkyl ether is produced as a by-product, the isomerization of olefin simultaneously occurs and so on.

It is ideal that allyl ether can be synthesized from alcohol and one mol amount of allyl alcohol without using an excessive additive and solvent. However, in reality, it is difficult to realize the efficient synthesis method by low desorption property of a hydroxyl group, and low nucleophilicity of alcohol.

A main object of the present invention, which has been developed in view of the problems pointed out above, is to provide a method of removing an allyl group that permits removing the allyl group in a single reaction step with a high reaction rate and a high functional group selectivity without using any additive, and a method of removing the allyl group by using the particular ruthenium complex. Furthermore, an object of the present invention is to provide a technology for synthesizing allyl ethers with a high efficiency by means of catalytic dehydrative allylation reaction from allyl alcohol and alcohol without using any additive.

As a result of an extensive research conducted in an attempt to achieve the object noted above, the present inventors have found that it is effective to use a complex of cyclopentadienyl ruthenium as a catalyst in the catalytic removal of the allyl group. The complex of "cyclopentadienyl ruthenium", which is hereinafter referred to as "CpRu" in some cases, noted above comprises as a ligand an organic acid having a nitrogen atom in the α-position. Specifically, it has been found that, in the case of using the CpRu complex noted above, the allyl group is markedly activated by the hydrogen bond so as to make it possible to carry out a reaction even with an alcohol having a very low nucleophilicity and, thus, to remove efficiently the allyl group in a single reaction step, arriving at the present invention.

As a result of having diligently researched and studied on the basis of the knowledge that the synthesis of allyl ether can be performed by means of catalytic dehydration type allylation reaction if a π allyl complex can be easily formed even in the case where an inactive allyl alcohol is used, the present inventors also have found that target allyl ether can be obtained with a high yield only when a trace of the above-described catalyst CpRu is added to the reaction system, and have completed the present invention.

According to a first aspect of the present invention, there is provided a cyclopentadienyl ruthenium (II) complex or (iv) complex having an α-imino acid type ligand or an α-amino acid type ligand.

In a first embodiment of the present invention, it is desirable for the α-imino acid type ligand to be selected from the group consisting of quinaldic acid and picolinic acid and for the α-amino acid type ligand to be provided by proline.

According to another aspect of the present invention, there is provided a method of removing the allyl group from allyl ethers, allyl carbonic acid esters and allyl esters comprising removing the allyl group in a solvent containing an alcohol in the presence of cyclopentadienyl ruthenium (II) complex or (iv) complex used as a catalyst.

The allyl ethers handled in the embodiment of the present invention is represented by general formula (I) or (II) given below:

R¹O-R (I)

where R denotes an allyl group, which may be substituted, and R¹ denotes C₆H₅CH₂CH₂, 2-indanyl, C₆H₅CH₂ (CH₃)₂C, C₆H₅, CH₂=CHCH₂CH₂CH₂, or HC≡ CCH₂CH₂CH2; 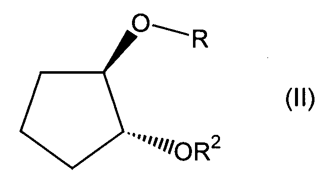 where R denotes an allyl group, which may be substituted, and R² denotes C₆H₅CO, C₆H₅CH₂, CH₃OCH₂, or (tert-C₄H₉) (C₆H₅) ₂Si.

Moreover, according to the present invention, a method of synthesizing allyl ethers, wherein allyl ethers are synthesized by means of dehydration type allylation reaction from the mixture of allyl alcohols and alcohols without using a solvent or in a non-protic solvent in the presence of the above-described cyclopentadienyl ruthenium (II) complex or (IV) complex.

In an aspect of the present invention, the non-protic solvent contains at least one selected from dichloromethane, dichloroethane, chloroform, cyclopentylmethylether, toluene, anisole and methyl acetate.

Moreover, in another aspect of the invention, the alcohol is selected from 2-phenylethanol, cyclohexanol, 2-indanol, 1,1-dimethyl-2-phenylethanol, 3-butenol, 5-hexenol, 4-pentynol, phenol and geraniol.

According to the present invention, in the removal of the allyl series protecting group and in the synthesizing of the allyl ethers, a novel ruthenium complex which indicates a high reactivity has been provided under the mild condition. Moreover, the technology for efficiently removing the allyl group and the technology for efficiently synthesizing allyl ethers which do not require an excessive additive except that catalyst is added have been provided. These technologies provided by the present invention sufficiently satisfy a high reactivity and functional group selectivity, the simplicity of the process, cost effectiveness, environmental harmony and the like, thus being extremely industrially effective.

This summary of the invention does not necessarily describe all necessary features so that the invention may also be a sub-combination of these described features.

The novel ruthenium complex provided by the present invention, which is used as a catalyst, is a CpRu(II) complex or (IV) complex having an α-imino acid type ligand or an α-amino acid type ligand (hereinafter also referred to as "CpRu complex of the present invention"). The CpRu(II) complex of the present invention exhibits a highly excellent catalytic function for the removal of the allyl group acting as a protecting group of, for example, the hydroxyl group.

The present inventors have found that, in the removal of the allyl group by using the CpRu(II) or (IV) catalyst in an solvent, the allyl group can be effectively removed by simply carrying out the reaction of the substrate in an alcohol based solvent under the atmospheric pressure in the case of using a novel complex comprising an organic acid having a nitrogen atom in the α-position. To be more specific, where the substrate is formed of allyl ethers, a hydrogen bond is formed in the presence of the novel catalyst between the oxygen atom in the allyl ether portion of the substrate and the hydrogen atom in the acid portion of the ligand in the substrate-catalyst complex formed in the alcohol based solvent, with the result that the nucleophilicity of the allyl group is improved by the hydrogen bond. On the other hand, the nucleophilicity of the Ru ion is enhanced by the coordination of Cp having the electron donor nature with the sp² nitrogen atoms, with the result that the Ru ion of the catalyst is allowed to carry out the nucleophilic reaction so as to produce a catalytic function excellent for the removal of the allyl group. Incidentally, the term "alcohol based solvent" used herein represents an alcohol solvent or a mixed solvent containing alcohol.

In the present invention, an α-imino acid type ligand or an α-amino acid type ligand is used as an organic acid ligand having a nitrogen atom in the α-position. Particularly, the α-imino acid type ligand, which exhibits a higher activity, is suitable for use in the present invention. To be more specific, the organic acid having a nitrogen atom in the α-position, which is used in the present invention, is selected from the group consisting of quinaldic acid and picolinic acid. It is particularly desirable to use quinaldic acid as the organic acid having a nitrogen atom in the α-position. Also, the α-amino acid type ligand used in the present invention includes, for example, proline. The method of synthesizing the CpRu(II) complex or (IV) complex of the present invention is not particularly limited. For example, the CpRu(II) complex of the present invention can be synthesized by mixing [CpRu(CH₃CN)₃]PF₆ and an α-imino acid or an α-amino acid in an alcohol based solvent. The solvent used is not particularly limited as far as the solvent permits dissolving [CpRu(CH₃CN)₃]PF₆ and an α-imino acid or an α-amino acid.

In the case of using the CpRu(II) complex or (IV) complex of the present invention, it is possible to remove the allyl group with a high reaction rate by simply carrying out the reaction in an alcohol based solvent under the atmospheric pressure without using any additive. It is difficult to remove the allyl group from the allyl compounds, particularly allyl ethers. However, the CpRu(II) complex or (IV) complex of the present invention exhibits a catalytic function highly excellent in respect of the allyl ethers.

In the removing method of the allyl group according to one embodiment of the present invention, the allyl group is removed from the allyl compounds such as allyl ethers and allyl carbonic acid esters in an alcohol based solvent in the presence of the CpRu complex of the present invention. The removing method can be performed under an inert gas atmosphere such as an argon gas atmosphere or a nitrogen gas atmosphere under the atmospheric pressure.

The alcohol based solvent used in the present invention is not particularly limited as far as the solvent is capable of dissolving the substrate and the CpRu complex. To be more specific, it is possible to use an alcohol solvent such as methanol, ethanol, or isopropyl alcohol singly or in the form of a mixture of a plurality of these alcohols. It is also possible to use, for example, a halogen series solvent such as dichloromethane, an ether series solvent such as tetrahydrofuran (THF), an aromatic hydrocarbon series solvent such as benzene, and an amide series solvent such as dimethyl formamide (DMF) together with the alcohol solvent as far as the mixed solvent permits dissolving the substrate and the CpRu(II) complex of the present invention.

It is possible to set appropriately the reaction temperature and the reaction time in a range of between about 0°C and 100°C and in a range of between several minutes and several hours, respectively. Also, the substrate/catalyst ratio (molar ratio) can be set appropriately in accordance with, for example, the reaction temperature and the reaction time.

Specific examples of the allyl ethers providing the substrate include the compounds represented by general formula (I) or (II) given below:

R¹O-R (I)

where R denotes an allyl group, which may be substituted, and R¹ is selected from the group consisting of (1a) to 1(f) given below:
(1a) R¹: C₆H₅CH₂CH₂,
(1b) R¹: 2-indanyl,
(1c) R¹: C₆H₅CH₂(CH₃)₂C,
(1d) R¹: C₆H₅,
(1e) R¹: CH₂=CHCH₂CH₂CH₂,
(1f) R¹: HC≡CCH₂CH₂CH₂ ; 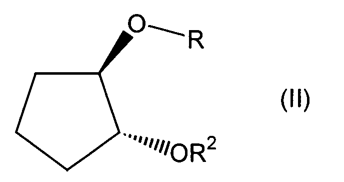 where R denotes an allyl group, which may be substituted, and R² is selected from the group consisting of (2a) to 2(d) given below:

(2a) R²: C₆H₅CO,
(2b) R²: C₆H₅CH₂,
(2c) R²: CH₃OCH₂,
(2d) R²: (tert-C₄H₉) (C₆H₅)₂Si

The technology of the present invention for removing the allyl group permits removing the allyl group with a high reaction rate. In addition, when it comes to the multifunctional compound represented by, for example, general formula (II) given above, it is possible to selectively remove the allyl group alone with a high selectivity.

The present inventors also have found that the catalytic function using the CpRu (II) complex or (IV) complex of the present invention also shows an excellent functional effect also in the generation of allyl ethers by utilizing dehydration type allylation reaction.

Specifically, in an embodiment of a method of manufacturing allyl ethers according to the invention, allyl ethers are manufactured by means of dehydration type allylation reaction from the mixture of allyl alcohols and alcohols without using a solvent in the presence of the CpRu complex of the invention. In another embodiment, allyl ethers are manufactured by means of dehydration type allylation reaction from the mixture of allyl alcohols and alcohols in a solvent using a non-protic solvent as the solvent in the presence of the above-described catalyst.

In the above method of removing the allyl group, the alcohol based solvent (protic solvent) is utilized, and in contrast to this, in the method of manufacturing allyl ethers, it is characterized in that the solvent is not used or the non-protic solvent is used in the catalytic reaction using the same catalyst. The non-protic solvent is not particularly limited, and dichloromethane, dichloroethane, chloroform, cyclopentylmethylether, toluene, anisole, methyl acetate, and the like can be singly used or two or more kinds of them can be used by mixing.

Alcohol which is a substrate can be selected in accordance with the kinds of allyl ethers as required. Examples of the alcohol include 2-phenylethanol, cyclohexanol, 2-indanol, 1,1-dimethyl-2-phenylethanol, 3-butenol, 5-hexenol, 4-pentynol, phenol, and geraniol. Moreover, the mixture ratio between allyl alcohol and alcohol is not particularly limited, and it can be appropriately set in accordance with the kinds of substrate or the like, but, it is preferable that the ratio is in the range from 1:1 to 1:2.

The reaction temperature and reaction time can be appropriately set in the range from nearby 0°C to 100°C and in the range from several minutes to several hours, respectively. In addition, substrate/catalyst ratio (molar ratio) can be appropriately set in accordance with the reaction temperature, the reaction time and the like.

### (Examples)

The present invention will now be described on the basis of some Examples. Needless to say, however, the technical scope of the present invention is not limited by the following Examples.

### 1. Deallylation reaction

### (Example 1)

Table 1 shows the results of the deallylation reaction of the ally ethers represented by general formula (I) given above in the case of using a [CpRu (CH₃CN)₃]PF₆-quinaldic acid composite system as the CpRu(II) complex of the present invention. The reaction was carried out in methanol at 30°C under the condition of [[CpRu (II) (CH₃CN)₃]PF₆] = [quinaldic acid] = 1 mM unless otherwise specified

**Table 1:**

| Deallylation reaction of allyl ethers catalyzed by [CpRu (CH₃CN)₃]PF₆-quinaldic acicomposite system | | | | | |
|---|---|---|---|---|---|
| No. | Substrate [mM] | S/C^{[a]} | Solvent | Time [h] | Yield [%] ^{[b]} |
| 1 | 1a(100) | 100 | CH₃OH | 0.5 | >99 |
| 2 | 1a (500) | 500 | CH₃OH | 3 | 99 |
| 3 | 1a (1000) | 1000 | CH₃OH | 3 | 98 ^{[c]} |
| 4 ^{[d]} ^{[e]} | 1a (1000) | 10000 | CH₃OH | 17 | 41 |
| 5 | 1a (100) | 100 | C₂H₅OH | 2 | 99 |
| 6 | 1a (100) | 100 | i-C₃H₇OH | 3 | 98 |
| 7 | 1a(100) | 100 | t-C₄H₉OH | 13 | 82 |
| 8 | 1a(100) | 100 | 1:1 CH₃OH-H₂O | 6 | 99 |
| 9 | 1a(100) | 100 | 1:1 CH₃OH-DMF | 6 | 99 |
| 10 | 1a(100) | 100 | 1:1 CH₃OH-THF | 0.5 | 99 |
| 11 | 1a(100) | 100 | 1:1 CH₃OH-CH₂Cl₂ | 0.5 | 99 |
| 12 | 1b(500) | 500 | CH₃OH | 3 | 99 |
| 13 | 1c(500) | 500 | CH₃OH | 3 | >99 |
| 14 | 1d(100) | 100 | CH₃OH | 3 | >99 |
| 15 | 1e(500) | 500 | CH₃OH | 3 | 97 |
| 16 | 1f(500) | 500 | CH₃OH | 3 | 94 |

| | | | | | |
|---|---|---|---|---|---|
| [a]S/C=Substrate/catalyst ratio, | | | | | |
| [b]GC analysis, | | | | | |
| [c]200mmHg Ar, | | | | | |
| [d] 70C°, | | | | | |
| [e] [catalyst] =0.1mM | | | | | |

As shown in Table 1, the reaction rate is very high in the case of using a [CpRu(CH₃CN)₃]PF₆-quinaldic acid composite system as the catalyst even if the substrate is formed of allyl ethers. When it comes to sample No. 1 shown in Table 1, the reaction was finished within 30 minutes. The removal of the allyl group was achieved in sample No. 3 under the reaction temperature of 30°C even where the substrate/catalyst ratio was 1,000. The allyl group can be removed even if the substrate/catalyst ratio is increased 10,000, if the reaction temperature is set at 70°C, as apparent from sample No. 4. Alcohols other than methanol such as ethanol and isopropyl alcohol can also be used as the solvent as in samples Nos. 5 and 6. However, where tert-butyl alcohol, which has a solubility of the catalytic system lower than that of, for example, methanol, is used as the solvent, the yield is somewhat lowered, as apparent from sample No. 7. Further, samples Nos. 8 to 11 support that each of water, DMR, THF, and dichloroethane can be used for forming a mixed solvent together with methanol.

The deallylation was achieved with a high reaction rate in each of the primary, secondary and tertiary alkyl ethers of 1a, 1b, 1c (samples Nos. 2, 12 and 13) and the ally phenyl ether 1d (sample No. 14). Allyl 4-pentenyl ether (1e) can be converted into 4-pentenol without involving any olefin isomerization, as apparent from sample No., 15. Further, the yield is slightly lowered in the reaction using acetylene-containing alkyl allyl ether (1f) . However, a high yield of 94% is retained even in this case as apparent from sample No. 16.

### (Example 2)

The functional group selectivity in the allyl group removal in the present invention was studied by using (±)-trans-1,2-cyclopentane diols represented by general formula (II). It should be noted that one of the hydroxyl groups is protected as allyl ether, and the other hydroxyl group is protected as benzoic acid ester (2a), benzyl ether (2b), methoxy methyl ether (2c), or tert-butyl phenyl silyl ether (2d). In each of all the cases, the allyl group was selectively removed with a high yield exceeding 99% ([2a~d] = 100 mM, [catalyst] = 1 mM, 30°C, 0.5 ~ 4 hours).

### (Example 3)

[CpRu(CH₃CN)₃]PF₆ (27 mg, 62 µmol) and methanol (5.5 mL) were placed in a 20-mL Schlenk tube under an argon stream. A 100 mM methanol solution of quinaldic acid (0.62 mL, 62 µmol) was added to the mixture. After the mixture was allowed to stand for 30 minutes at 24°C, the reddish brown solution was transferred into a 150-mL Schlenk tube equipped with Young's bulb containing 2-phenyl ally ether (5.0g, 31 mmol) and methanol (51 mL), and the resultant mixture was kept stirred at 30°C for 3 hours. As a result of the GC analysis, it was found that 2-phenyl ethanol was obtained at a yield of 99% (the conditions were: capillary column, J&W Scientific DB-WAX (0.25 mm X 15 m); column temperature, 50 to 150°C; temperature elevation rate, 10°C/min; detection temperature, 250°C; carrier gas, He; column pressure, 118 kPa; split ratio, 100:1). The reaction mixture was concentrated under a reduced pressure so as to give a crude product. The crude product thus obtained was distilled (55°C/0.01 mmHg) so as to obtain a pure 2-phenyl ethanol at a yield of 98%.

### 2. Method of catalytic dehydration type synthesizing allyl ether

### (Example 4) Non-solvent reaction system

2-phenylethanol was used as a standard substrate, and by utilizing two kinds of catalysts of the present invention comprising one mol amount of allyl alcohol, 1/2000 molar quantity of [CpRu(CH₃CN)₃]PF₆ and 2-quinolinecarboxylic acid (quinaldic acid) or 2-pyridine carboxylic acid (picolinic acid), the generation amount of allyl 2-phenylethylether after 30 minutes under the temperature of 70°C was measured by GC analysis (capillary column, J & W Scientific DB-WAX (0.25 mm x 15 m); column temperature, 50 to 250°C; rising temperature rate, 10°C/min; t_{R} (2-phnylethanol) 6.0 min; t_{R} (allyl 2-phenylethyl ether) 4.0 min; detection temperature, 250°C; carrier gas, helium; pressure of column, 50kPa; flow rate, 3.5 mL/sec). The results are indicated in Table 2. In any one of the cases where catalyst was used, considerable high activity was indicated. Under the present conditions, particularly the activity in the case of using catalyst containing 2-quinolinecarboxylic acid as a ligand was high, the yield under the reaction conditions became 66%, and after 6 hours, the yield reached to 99% (No. 1).

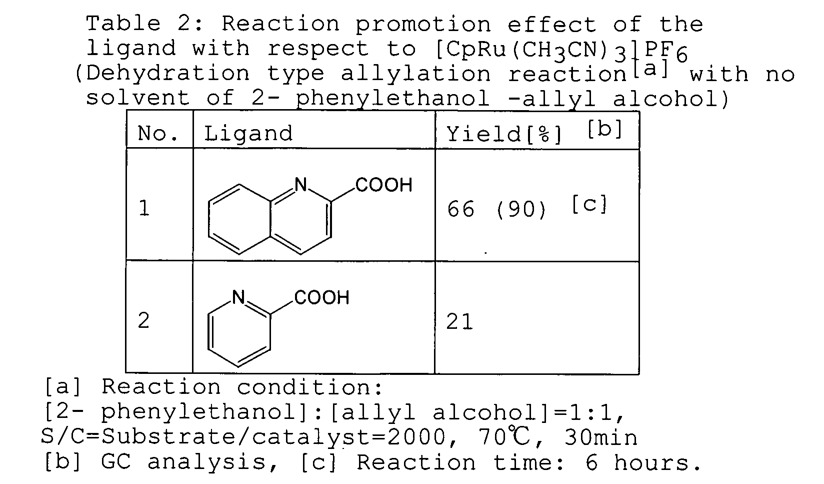

Next, 2-quinolinecarboxylic acid indicating a high activity was used as a ligand, and the reaction conditions were optimized. The results are shown in Table 3.

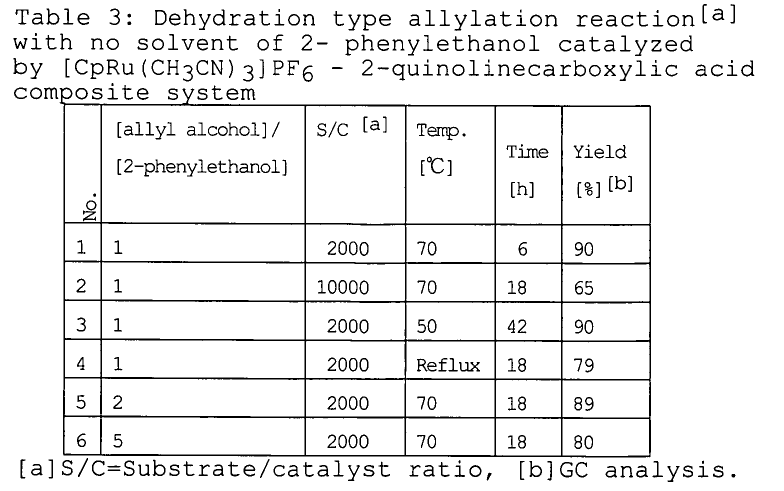

### (Example 5) Reaction system using non-protic solvent

Table 4 shows the results of the reaction promotion effect of the ligand with respect to [CpRu(CH₃CN)₃]PF₆ in the case where dichloromethane was used as a non-protic solvent. One mol amount of allyl alcohol was used under the conditions of substrate concentration 100 mM, substrate catalyst ratio 100, and under the refluxing, the yields of the product after 30 minutes were compared. In the present conditions, similarly to the case of non-solvent conditions, the activity of 2-quinolinecarboxylic acid is higher in 5-50 fold in comparison with those of 1-isoquinolinecarboxylic acid, 3-isoquinolinecarboxylic acid, and 2-pyridine carboxylic acid (Nos. 1 to 4). When the carboxyl group of 2-pyridine carboxylic acid was replaced with the hydroxymethyl group and the carbomethoxyl group, the activity was disappeared (Nos. 5 and 6). Similarly, also in 2-(aminomethyl)pyridine and 2-pyridine carboxylic acid sodium salt, the activity was not indicated in dichloromethane at all (Nos. 7 and 8). The activity of piperidine carboxylic acid has not found (No. 9). Diphenylphosphinoacetic acid gives 1,1-di (2-phenylethoxy) propane considered to be derived from a 1,3-hydrogen shift generation product (No. 10).

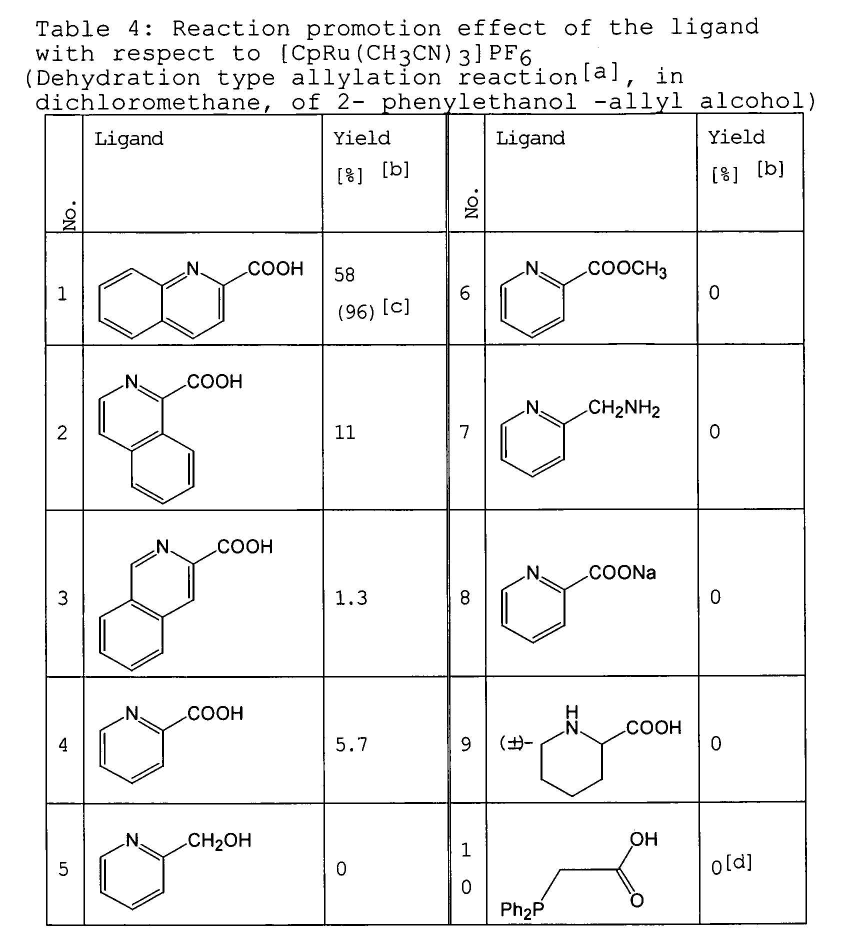

Next, 2-quinolinecarboxylic acid indicating the high activity was used as a ligand, and the reaction conditions were optimized. The results are shown in Table 5.

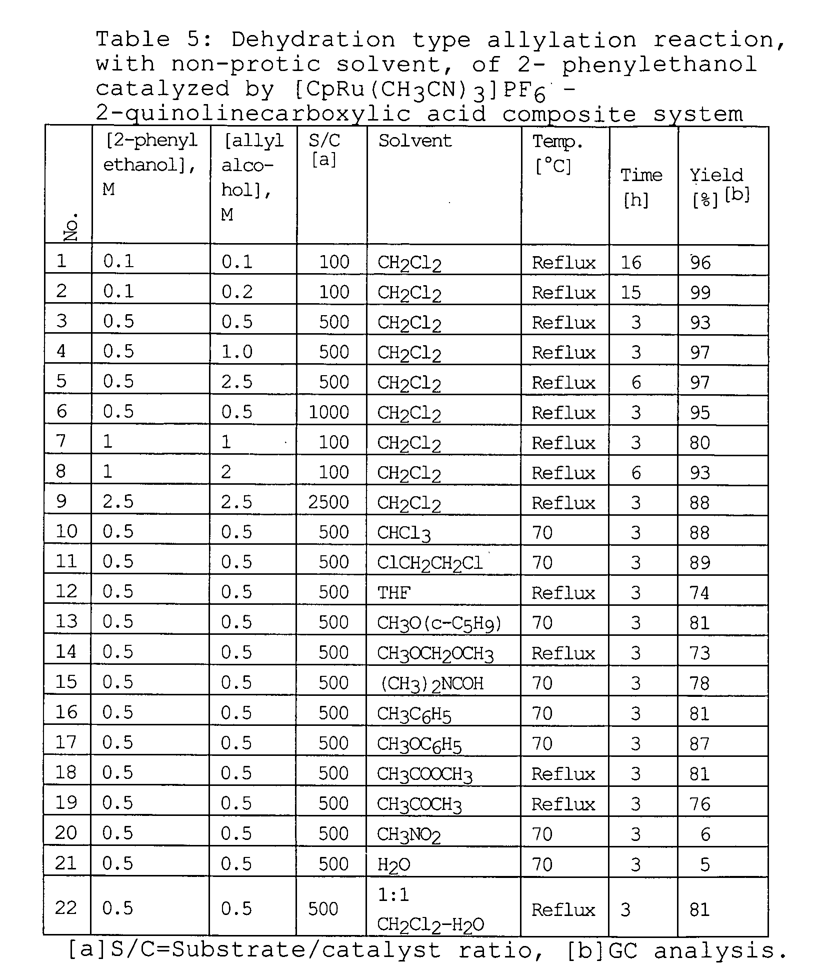

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A cyclopentadienyl ruthenium (II) complex or (iv) complex having an α-imino acid type ligand or an α-amino acid type ligand.

2. The cyclopentadienyl ruthenium (II) complex or (iv) complex according to claim 1, **characterized in that** the α-imino acid type ligand is selected from the group consisting of quinaldic acid and picolinic acid.

3. The cyclopentadienyl ruthenium (II) complex or (iv) complex according to claim 1 or 2, **characterized in that** the α-amino acid type ligand is a proline.

4. A method of removing the allyl group from allyl ethers, allyl carbonic acid esters and allyl esters, **characterized by** comprising removing the allyl group in a solvent containing an alcohol in the presence of the cyclopentadienyl ruthenium (II) complex or (iv) complex according to claim 1 or 2.

5. A method of removing the allyl group from allyl ethers, allyl carbonic acid esters and allyl esters, **characterized by** comprising removing the allyl group in a solvent containing an alcohol in the presence of the cyclopentadienyl ruthenium (II) complex or (iv) complex according to claim 3.

6. The method of removing the allyl group according to claim 4, **characterized in that** the allyl ethers are represented by general formula (I) or (II) given below:
R¹O-R (I)
where R denotes an allyl group, which may be substituted, and R¹ denotes C₆H₅CH₂CH₂, 2-indanyl, C₆H₅CH₂(CH₃)₂C, C₆H₅, CH₂=CHCH₂CH₂CH₂, or HC≡ CCH₂CH₂CH₂; 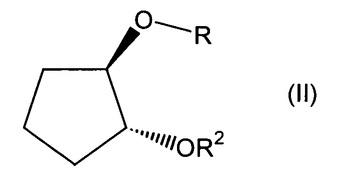 where R denotes an allyl group, which may be substituted, and R² denotes C₆H₅CO, C₆H₅CH₂, CH₃OCH₂, or (tert-C₄H₉) (C₆H₅)₂Si.

7. The method of removing the allyl group according to claim 5, **characterized in that** the allyl ethers are represented by general formula (I) or (II) given below:
R¹O-R (I)
where R denotes an allyl group, which may be substituted, and R¹ denotes C₆H₅CH₂CH₂, 2-indanyl, C₆H₅CH₂(CH₃)₂C, C₆H₅, CH₂=CHCH₂CH₂CH₂, or HC≡ CCH₂CH₂CH₂; 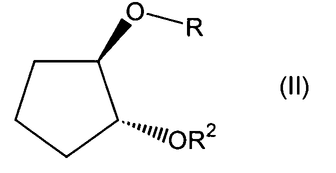 where R denotes an allyl group, which may be substituted, and R² denotes C₆H₅CO, C₆H₅CH₂, CH₃OCH₂, or (tert-C₄H₉) (C₆H₅) ₂Si .

8. A method of synthesizing allyl ethers, **characterized in that** allyl ethers are synthesized by means of dehydration type allylation reaction from the mixture of allyl alcohols and alcohols without using a solvent in the presence of the cyclopentadienyl ruthenium (II) complex or (IV) complex according to claim 1 or 2.

9. A method of synthesizing allyl ethers, **characterized in that** allyl ethers are synthesized by means of dehydration type allylation reaction from the mixture of allyl alcohols and alcohols without using a solvent in the presence of the cyclopentadienyl ruthenium (II) complex or (IV) complex according to claim 3.

10. A method of synthesizing allyl ethers, **characterized in that** allyl ethers are synthesized by means of dehydration type allylation reaction from the mixture of allyl alcohols and alcohols in a non-protic solvent in the presence of the cyclopentadienyl ruthenium (II) complex or (IV) complex according to claim 1 or 2.

11. A method of synthesizing allyl ethers, **characterized in that** allyl ethers are synthesized by means of dehydration type allylation reaction from the mixture of allyl alcohols and alcohols in a non-protic solvent in the presence of the cyclopentadienyl ruthenium (II) complex or (IV) complex according to claim 3.

12. The method of synthesizing allyl ethers according to claim 10, **characterized in that** the non-protic solvent contains at least one selected from dichloromethane, dichloroethane, chloroform, cyclopentylmethylether, toluene, anisole and methyl acetate.

13. The method of synthesizing allyl ethers according to claim 11, **characterized in that** the non-protic solvent contains at least one selected from dichloromethane, dichloroethane, chloroform, cyclopentylmethylether, toluene, anisole and methyl acetate.

14. The method of synthesizing allyl ethers according to claim 8, **characterized in that** the alcohol is selected from 2-phenylethanol, cyclohexanol, 2-indanol, 1,1-dimethyl-2-phenylethanol, 3-butenol, 5-hexenol, 4-pentynol, phenol and geraniol.

15. The method of synthesizing allyl ethers according to claim 9, **characterized in that** the alcohol is selected from 2-phenylethanol, cyclohexanol, 2-indanol, 1,1-dimethyl-2-phenylethanol, 3-butenol, 5-hexenol, 4-pentynol, phenol and geraniol.

16. The method of synthesizing allyl ethers according to claim 10, **characterized in that** the alcohol is selected from 2-phenylethanol, cyclohexanol, 2-indanol, 1,1-dimethyl-2-phenylethanol, 3-butenol, 5-hexenol, 4-pentynol, phenol and geraniol.

17. The method of synthesizing allyl ethers according to claim 11, wherein the alcohol is selected from 2-phenylethanol, cyclohexanol, 2-indanol, 1,1-dimethyl-2-phenylethanol, 3-butenol, 5-hexenol, 4-pentynol, phenol and geraniol.

18. The method of synthesizing allyl ethers according to claim 12, **characterized in that** the alcohol is selected from 2-phenylethanol, cyclohexanol, 2-indanol, 1,1-dimethyl-2-phenylethanol, 3-butenol, 5-hexenol, 4-pentynol, phenol and geraniol.

19. The method of synthesizing allyl ethers according to claim 13, **characterized in that** the alcohol is selected from 2-phenylethanol, cyclohexanol, 2-indanol, 1,1-dimethyl-2-phenylethanol, 3-butenol, 5-hexenol, 4-pentynol, phenol and geraniol.
